# EUROPEAN PATENT APPLICATION

(11) **EP 2 886 046 A1**
(43) Date of publication of application: **24.06.2015**
(21) Application number: 14196856.0
(22) Date of filing: 09.12.2014
(51) Int. Cl.: A61B 5/00, A61B 5/044, A61B 5/0452

(54) **Bio-information display device and bio-information display method**

(30) Priority: 20.12.2013 JP 2013263679
(71) Applicant: Nihon Kohden Corporation, Shinjuku-ku Tokyo (JP)
(72) Inventor: Mato, Takashi, Meguro-ku, Tokyo (JP); Matsueda, Hideyo, Kawagoe-shi, Saitama (JP); Kaiami, Takashi, Shinjuku-ku, Tokyo (JP); Takayanagi, Tsuneo, Shinjuku-ku, Tokyo (JP)
(74) Representative: Grünecker Patent- und Rechtsanwälte PartG mbB

(57) **Abstract**

A bio-information display device includes a calculator that calculates a bio-parameter value from a biosignal, a graph generator that generates a trend graph based on the bio-parameter value, a waveform generator that generates a signal waveform from the biosignal, a display that displays information, and a display controller that synchronizes the bio-parameter value of the trend graph with the signal waveform associated with the bio-parameter value and then sequentially displays the trend graph and the signal waveform on the display in time-series.

## Description

### BACKGROUND

The present invention relates to a bio-information display device and a bio-information display method, which allow a careful examination of a bio-information to be quickly and accurately performed.

Conventionally, bio-information, such as electrocardiogram is used in understanding a condition of a patient or detecting an abnormality thereof. In this case, variations in both of a trend graph of a parameter value associated with the bio-information and signal waveforms over time have to be visually observed. Specifically, the signal waveforms have to be examined while being compared one by one.

However, when a bio-information is examined over a long period of time, data thereof becomes a large amount, and therefore such examination takes a long time and is not efficient.

Accordingly, Japanese Patent No. 3154425 discloses waveforms corresponding to a trend of measured value are overlapped with each other and then a measurement is performed by arranging side by side the overlapped waveforms during a certain period of time (every 30 seconds).

According to the above device, it would be believed that a condition of a patient or an abnormality thereof can be understood or detected from conditions of the overlapped waveforms. However, when a variation in a parameter value is not repeated, a variation in the overlapped waveforms is rarely exhibited and thus likely to be often overlooked. In addition, with respect to data over a long period of time, there is a problem in that a long time is taken to examine the overlapped waveforms. For example, considering data for one hour, waveforms corresponds to 120 screens when one screen is displayed at 30 second intervals and also, considering data for ten hours, waveforms corresponds to 1200 screens when one screen is displayed at 30 second intervals. As a result, a burden on an examiner and an extended examination time cannot be avoided.

### SUMMARY

The present invention has been made keeping in mind the current situation of the bio-information examination as described above, and an object thereof is to provide a bio-information display device, which allows a careful examination of a bio-information to be quickly and accurately performed.
(1) According to an aspect of the invention, a bio-information display device, includes:
   a calculator that calculates a bio-parameter value from a biosignal;
   a graph generator that generates a trend graph based on the bio-parameter value;
   a waveform generator that generates a signal waveform from the biosignal;
   a display that displays information; and
   a display controller that synchronizes the bio-parameter value of the trend graph with the signal waveform associated with the bio-parameter value and then sequentially displays the trend graph and the signal waveform on the display in time-series.
(8) According to another aspect of the invention, a bio-information display method for displaying on a display a trend graph based on a bio-parameter calculated from a biosignal and a signal wave form generated from the biosignal, includes:
   displaying the trend graph on a first region of the display;
   displaying a cursor which indicate a part of the trend graph and is movable along the trend graph; and
   displaying a signal waveform associated with the part of the trend graph indicated by the cursor on a second region of the display.
(2) In the bio-information display device of (1), the display controller changes a display aspect of the signal waveform, depending on a magnitude of variation in the bio-parameter value over time, and then displays the signal waveform on the display according to the changed display aspect.
(3) In the bio-information display device of (2), the display controller changes, as the display aspect to be changed, a display time of the signal waveform and then displays the signal waveform on the display according to the changed display time.
(4) In the bio-information display device of (3), the display controller displays the signal waveform on the display in such a manner that the display time is set to be longer if a variation in the bio-parameter value over time is large and the display time is set to be shorter if a variation in the bio-parameter value over time is small.
(5) In the bio-information display device (3) or (4), wherein the display controller displays the signal waveform on the display in such a manner that displaying is stopped during a predetermined period of time if a variation in the bio-parameter value over time is large.
(6) In the bio-information display device of any one of (1) to (5), the display controller displays the signal waveform on the display in such a manner that a reference signal waveform is overlapped with or close to the signal waveform.
(7) In the bio-information display device of any one of (1) to (6), the biosignal is an electrocardiogram signal.

The bio-information display device according to the configurations (1) to (8) can synchronize the bio-parameter value of the trend graph with the signal waveform associated with the bio-parameter value and then sequentially display the trend graph and the signal waveform on the display in time-series. Therefore, when an abnormal value or the like with respect to the bio-parameter value exists, a signal waveform displayed synchronizing thereto can be immediately recognized, thereby allowing a careful examination to be accurately performed.

### BRIEF DESCRIPTION OF THE DRAWINGS

Fig. 1 is a configuration view illustrating an embodiment of a bio-information display device according to the present invention.
Fig. 2 is a waveform view illustrating a basic electrocardiogram waveform and parameters.
Fig. 3 is a view illustrating a display example of a case where a trend graph and signal waveforms are displayed to be synchronized with each other by the embodiment of the bio-information display device according to the invention.
Fig. 4 is a view illustrating a display example of a case where the signal waveform are displayed to be overlapped with a reference signal waveform by the embodiment of the bio-information display device according to the invention.

### DETAILED DESCRIPTION OF EMBODIMENTS

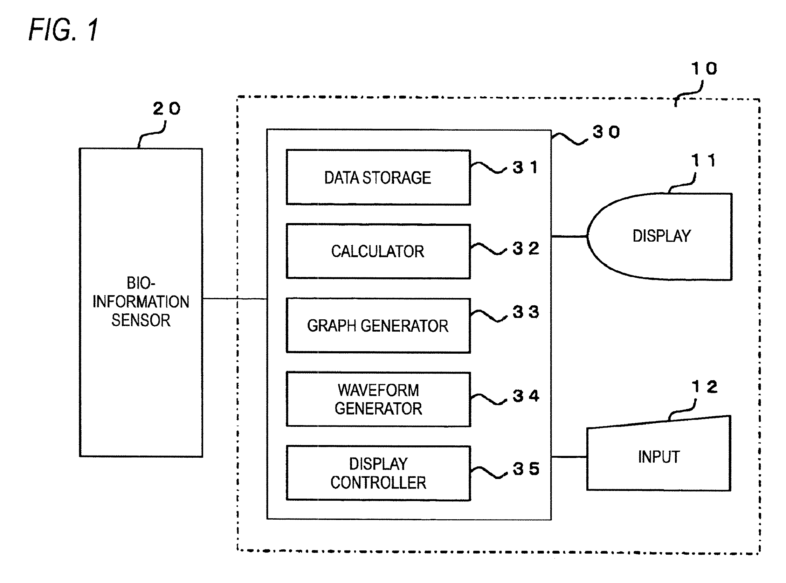

Embodiments of a bio-information display device according to the present invention will be now described with reference to the accompanying drawings. Fig. 1 is a configuration view illustrating an embodiment of a bio-information display device according to the invention. The bio-information display device 10 is configured to be mounted on an information measuring apparatus (not shown), such as an electrocardiograph, an electroencephalograph, a pulse oximeter, or a bio-information monitor, and a bio-information sensor 20 used in the bio-information measuring apparatus is connected thereto.

The bio-information measuring device 10 has a main body 30, a display 11 as a display connected thereto, and an input unit 12 for inputting information or command. The display 11 is constituted by an LCD or the like. Also, the input unit 12 can be constituted by a keyboard, a mouse, or a touch panel provided on a screen of the display 11.

The main body 30 can include a computer having a CPU and a main memory or an external memory. The main body 30 is provided with a data storage 31 for storing, as bio-information data, digital biosignals created by sampling and A/D-converting biosignals obtained from the bio-information sensor 20. In this example, although signals obtained from the bio-information sensor 20 is stored in the data storage 31, digital biosignals sampled and A/D-converted by any other devices may be stored in the data storage 31.

The display 11, the input unit 12, the CPU, the memory and the like used herein are equipped in the bio-information measuring apparatus, but may be separately prepared. In addition, functions of the bio-information display device 10 may be also embodied by connecting a general-purpose computer, such as PC, to the bio-information measuring apparatus and using data stored in the data storage 31.

The main body 30 is additionally provided with a calculator 32, a graph generator 33, a waveform generator 34 and a display controller 35. The calculator 32 is intended to calculate bio-parameter values (described below) from the biosignal data stored in the data storage unit 31. The graph generator 33 is intended to generate a trend graph based on the bio-parameter values. The waveform generator 34 is intended to generate a signal waveform from the biosignal data.

The display controller 35 synchronizes a trend graph of bio-parameter values, which is generated by the graph generator 33, with a signal waveform, which is generated by the waveform generator 34, associated with the bio-parameter values, and then sequentially displays the trend graph and the signal waveform on the display 11, which is a display, in time-series. Upon displaying, the display controller 35 is intended to change a display aspect of the signal waveform depending on magnitudes of variation in bio-parameter values over time and then to display the signal waveform according to the changed display aspect. Hereinafter, although changing of the display aspect will be described as changing of a display time, a display color may be changed. As used herein, the phrase 'changing of a display time' unit changing of a period of time taken until displaying of signal waveforms at a certain point in time is switched to displaying of signal waveform at the next point in time.

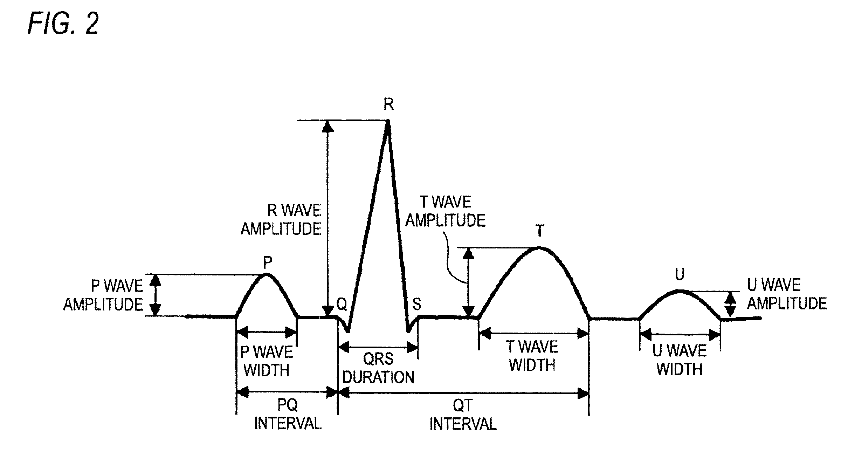

Next, an electrocardiogram signal as a biosignal will be described as one example. As shown in Fig. 2, parameters associated with the electrocardiogram signal includes QRS duration, R wave amplitude, QT interval, QTc (corrected QT interval: a value obtained by correcting the QT interval by a hear rate) and the like. Herein, the hollowing description will be described in terms of QTc. Meanwhile, the parameters are naturally different depending on the type of the biosignals and thus can be properly selected depending on each biosignal.

The electrocardiogram signal continuously detected by the bio-information sensor 20 (electrocardiogram electrodes) is subjected to a processing, such as A/D conversion and then stored, as electrocardiogram data, in the data storage 31. The calculator 32 calculates QTc as a bio-parameter from the electrocardiogram data stored in the data storage 31 and then stores the QTc in the data storage 31.

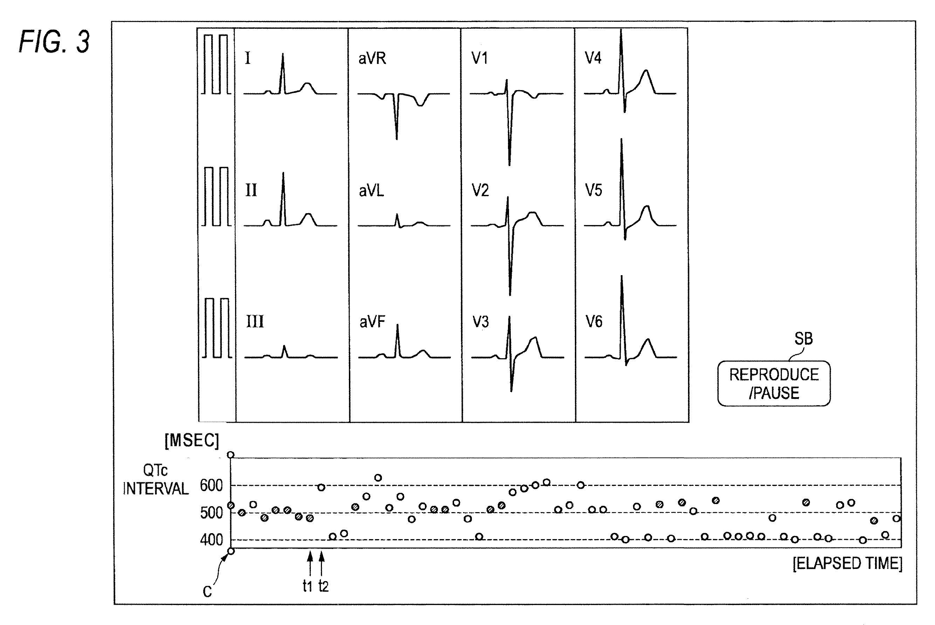

The graph generator 33 generates a trend graph of the QTc and the trend graph is displayed on the display 11 by the display controller 35. For example, the displayed trend graph is a graph displayed by plot points as shown in a lower area of Fig. 3. In this graph, a vertical axis indicates values of the QTc and a horizontal axis indicate time.

The waveform generator 34 generates electrocardiogram waveforms associated with the plot points of the trend graph, and the electrocardiogram waveforms are sequentially displayed in time-series on the display 11 by the display controller 35, synchronizing with the plot points of the trend graph. For example, the displayed electrocardiogram waveforms are displayed in an upper area located above the trend graph in Fig. 3. In Fig. 3, a standard 12-lead electrocardiogram waveform is shown.

Next, displaying of the trend graph of QTc and the electrocardiogram waveforms will be described in detail. In Fig. 3, a symbol SB is a reproducing/pause button and a symbol C is a cursor. If the reproducing/pause button SB is operated while the reproduce/pause button SB is indicated as a reproducing button, the indication is switched to a pause button and at the same time, the cursor C at a left end of the trend graph in the lower area is moved toward in a right direction at a predetermined speed. When the cursor C is coincided with a plot point of the QTc, an electrocardiogram waveforms associated with the QTc are displayed on the upper area. In Fig. 3, electrocardiogram waveforms when the cursor C is coincided with a plot point at the left end of the trend graph are shown. Displaying of the electrocardiogram waveforms is continued until the cursor C is coincided with the next plot point.

A period of time, during which the electrocardiogram waveforms is displayed, is changed depending on variations in the bio-parameter value QTc over time. In particular, values of the current QTc and the last QTc with respect to the QTc are compared with each other and if a variation amount, such as a differences therebetween, exceeds a predetermined threshold, the displaying time is set to become longer, but if the variation amount, such as differences therebetween is within the predetermined threshold, the displaying time is set to become a predetermined period of time shorter than that when exceeding the predetermined threshold.

In Fig. 3, for example, the QTc when the cursor C has reach a plot point at a time t2 is slightly less than 600, but is greatly increased from a slightly less than 500 at a plot point of a time t1 just before the time t2, and the variation amount in this case is considered as exceeding the predetermined threshold. Therefore, movement of the cursor C after the time t2 becomes slow and thus electrocardiogram waveforms at the time point can be unhurriedly observed in detail, so that whether or not there is an abnormality can be easily determined.

When wanting to observe in more detail, if the reproducing/pause button SB indicated as the pause button is operated, the cursor C continues to stop until the reproducing/pause button SB is again operated, and thus, the electrocardiogram waveforms can be sufficiently observed.

In the foregoing, although, as the display aspect of the electrocardiogram waveforms, if the variation amount in the bio-parameter value QTc exceeds the predetermined threshold, the display time is set to become longer and if being within the predetermined threshold, the displaying time is set to become a predetermined period of time shorter than that when exceeding the predetermined threshold, various aspects as the display aspect can be considered as follows.
(a) If the variation amount in QTc exceeds the threshold, displaying of the electrocardiogram waveforms is stopped during a predetermined period of time.
(b) A plurality of thresholds are prepared and the display time can be changed in three or more stages,
(c) The display time can be continuously (proportionally) changed depending on the variation amount in QTc.

Also, when calculating the variation amount in the bio-parameter value QTc, the variation amount may be calculated by a statistical process, such as comparison of the current QTc and the average (or maximum or minimum) value of a plurality of continuous QTc values including the last QTc adjacent thereto, or the like, rather than comparison of two adjacent QTc values.

In addition, when calculating the variation amount in the bio-parameter value QTc, a value, such as a difference between the current QTc and a normal value of QTc or a range in a normal range thereof may be obtained and the display time may be changed depending on the value, such as the difference. Namely, as the QTc is closer to the normal value or the normal range, the display time is set to become shorter, thereby quickly sending the electrocardiogram, whereas as the QTc is more deviated from the normal value or the normal range, the display time is set to become longer. By doing so, as the QTc is more deviated from the normal value or the normal range, the electrocardiogram waveforms can be further sufficiently observed. As a result, whether or not there is an abnormality can be easily found.

In the foregoing, although the cursor is used to specify the bio-parameter value QTc, plot points of QTc may adapted to sequentially be lightened. Also, in the foregoing, although the cursor is automatically moved by operating the reproducing/pause button SB, a separate manual reproducing button may be provided and thus the cursor or lightening of the plot points may be adapted to be moved only when the manual reproducing button is operated.

Although electrocardiogram waveforms corresponding to 12 leads are shown in the upper area of Fig. 3, the number of leads is only an example. For example, when diagnosing a certain disease, electrocardiogram waveforms, which can remarkably exhibit the disease, may be only displayed.

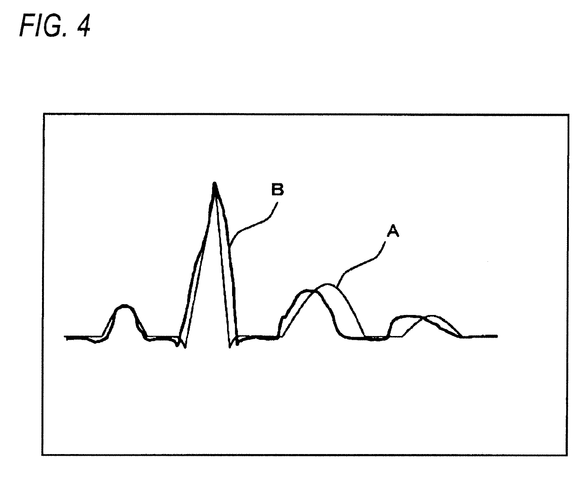

A reference electrocardiogram waveform may be displayed to be overlapped with or close to the electrocardiogram waveforms. For example, if a reference electrocardiogram waveform (in this example, a normal waveform of the associated patient) at any one lead is A in Fig. 4, the reference electrocardiogram waveform A can be overlapped with an actually measured electrocardiogram waveform B at the lead to be displayed as in Fig. 4. The actually measured electrocardiogram waveform B is likely to cause a variation in waveform over time, whereas the reference electrocardiogram waveform A has the same waveform regardless of time elapsed. Thus, due to such overlapping, a difference from the reference electrocardiogram waveform A can be recognized at a glance, and as a result, detection of abnormality can be more accurately performed. Although such an overlapped case is shown in Fig. 4, the actually measured electrocardiogram waveform may be displayed to be close to the reference electrocardiogram waveform.

In waveforms corresponding to 12 leads shown in Fig. 3, a display as in Fig. 4 is displayed on a screen portion for each lead. The reference electrocardiogram waveform may be generated by statistically processing normal electrocardiogram waveforms of several people, and also generated by statistically processing normal electrocardiogram waveforms of a respective patient. In addition, a normal waveform is typically used as the reference electrocardiogram waveform, but an abnormal waveform may be used as the reference electrocardiogram waveform.

In the bio-information display device according to the present invention as described above, a display time of electrocardiogram waveforms can be changed so that normal electrocardiogram waveforms can have a shorter display time, thereby achieving reduction in time. Also, when electrocardiogram waveforms, in which an abnormality is occurred, are displayed, the electrocardiogram waveforms can be displayed for a long time, and therefore overlooking can be reduced, thereby performing appropriate diagnoses. As a result, when the bio-information display device of the invention is employed in emergency medical care, this is very effective in that a condition of a patient, who has been transported, can be quickly and accurately diagnosed.

## Claims

1. A bio-information display device, comprising:
a calculator that calculates a bio-parameter value from a biosignal;
a graph generator that generates a trend graph based on the bio-parameter value;
a waveform generator that generates a signal waveform from the biosignal;
a display that displays information; and
a display controller that synchronizes the bio-parameter value of the trend graph with the signal waveform associated with the bio-parameter value and then sequentially displays the trend graph and the signal waveform on the display in time-series.

2. The bio-information display device according to claim 1, wherein the display controller changes a display aspect of the signal waveform, depending on a magnitude of variation in the bio-parameter value over time, and then displays the signal waveform on the display according to the changed display aspect.

3. The bio-information display device according to claim 2, wherein the display controller changes, as the display aspect to be changed, a display time of the signal waveform and then displays the signal waveform on the display according to the changed display time.

4. The bio-information display device according to claim 3, wherein the display controller displays the signal waveform on the display in such a manner that the display time is set to be longer if a variation in the bio-parameter value over time is large and he display time is set to be shorter if a variation in the bio-parameter value over time is small.

5. The bio-information display device according to claim 3 or 4, wherein the display controller displays the signal waveform on the display in such a manner that displaying is stopped during a predetermined period of time if a variation in the bio-parameter value over time is large.

6. The bio-information display device according to any one of claims 1 to 5, wherein the display controller Displays the signal waveform on the display in such a manner that a standard signal waveform is overlapped with or close to the signal waveform.

7. The bio-information display device according to any one of claims 1 to 6, wherein the biosignal is an electrocardiogram signal.

8. A bio-information displays method for displaying on a display a trend graph based on a bio-parameter calculated from a biosignal and a signal wave form generated from the biosignal, the method comprising:
displaying the trend graph on a first region of the display;
displaying a cursor which indicate a part of the trend graph and is movable along the trend graph; and
displaying a signal waveform associated with the part of the trend graph indicated by the cursor on a second region of the display.
